# EUROPEAN PATENT APPLICATION

(11) **EP 3 578 595 A1**
(43) Date of publication of application: **11.12.2019**
(21) Application number: 18747242.8
(22) Date of filing: 29.01.2018
(51) Int. Cl.: C08J 7/04, B32B 9/02, B32B 18/00, B32B 23/04, C01B 25/32, C08J 3/12

(54) **COMPOSITE BODY HAVING COATING LAYER OF CERAMIC CRYSTAL, AND METHOD FOR PRODUCING SAME**

(30) Priority: 02.02.2017 JP 2017017898; 27.04.2017 JP 2017087852; 07.09.2017 JP 2017171955
(71) Applicant: NATIONAL INSTITUTE OF ADVANCED INDUSTRIAL SCIENCE AND TECHNOLOGY, Chiyoda-ku Tokyo 100-8921 (JP)
(72) Inventor: NAGATA, Fukue, Nagoya-shi Aichi 463-8560 (JP); MIYAJIMA, Tatsuya, Nagoya-shi Aichi 463-8560 (JP); KATO, Katsuya, Nagoya-shi Aichi 463-8560 (JP)
(74) Representative: Mewburn Ellis LLP
(86) International application number: PCT/JP2018/002683
(87) International publication number: WO 2018/143120

(57) **Abstract**

Provided is a composite body where ceramic crystals are thinly and uniformly coated onto a base material containing a hydrophilic polymer. The composite body has a base material containing a hydrophilic polymer, and a ceramic layer which is coated onto at least a portion of a surface of the base material, and the ceramic layer contains crystals having a laminated structure. The ceramic layer may contain calcium phosphate. A thickness of the ceramic layer is 0.3 nm to 50 nm. The composite body is produced through a step of mixing and stirring a dispersion liquid in which the base material is dispersed, a first solution containing calcium ions, and a second solution containing phosphate ions.

## Description

### Technical Field

The present invention relates to a composite body having a base material containing a hydrophilic polymer and ceramic crystals coating the base material, and a method for producing the composite body.

### Background Art

A composite body is produced by coating a ceramic layer onto a base material. By coating the ceramic layer onto the base material, it is possible to impart chemical reaction resistance, chemical resistance, oxidation resistance, abrasion resistance, corrosion resistance and the like to the base material, and allows the base material to have higher breaking strength, higher toughness, higher resiliency, higher hardness, higher elasticity, higher gradient function and the like. When such a ceramic layer is thin, the composite body can satisfy both lightness and strength. Further, in the case where the ceramic layer of a composite body is made of calcium phosphate ceramic, the composite body is applicable to a material for absorption and separation of protein or the like, a filter which captures viruses or the like, a biosensor, a biocompatible material such as an artificial bone or an artificial tooth root and the like. In the case where a calcium phosphate layer which is coated onto a base material is formed of one continuous crystal layer, an optical function and internal stability of the composite body can be further enhanced. Recently, amidst the sophistication of a base material, a material where a ceramic layer is thinly and uniformly coated onto a surface of the base material has been attracting an attention.

To achieve the above-mentioned object, various methods for forming a ceramic film on a surface of a base material have been reported. For example, as such a film forming method, there has been known a plasma spraying method, a sputtering method, a flame spraying method, a baking method and the like can be named. However, these methods have a drawback that a base material is damaged by high temperature treatment. In view of the above, there has been a demand for a method which can thinly form a ceramic film at a relatively low temperature. Patent literature 1 describes a technique where a calcium phosphate-based compound layer is formed partially or wholly on a surface of an organic polymer base material. However, patent literature 1 merely describes that a needle-like or a plate-like calcium phosphate-based compound exists between the calcium phosphate-based compound layer and the organic polymer base material. That is, a thickness of the coating layer is not described in patent literature 1. Patent literature 2 describes a composite body where a core is made of a hydrophobic polymer and a shell is made of calcium phosphate.

### Citation List

### Patent Literature

PTL 1: JP 2003-253023 A
PTL 2: WO 2016/035750

### Summary of Invention

### Technical Problem

It is an object of the present invention to provide a composite body where ceramic crystals are thinly and uniformly coated onto a base material containing a hydrophilic polymer. Solution to Problem

The composite body according to the present invention has a base material containing a hydrophilic polymer, and a ceramic layer which is coated onto at least a portion of a surface of the base material, and the ceramic layer contains crystals having a laminated structure. A method for producing a composite body according to the present invention is a method for producing a composite body having a base material and a ceramic layer which is coated onto at least a portion of a surface of the base material, wherein the method has a step of mixing and stirring a dispersion liquid in which the base material is dispersed, a first solution containing calcium ions, and a second solution containing phosphate ions. Advantageous Effects of Invention

According to the present invention, it is possible to obtain a composite body where ceramic crystals are thinly and uniformly coated onto a base material containing a hydrophilic polymer.

### Brief Description of Drawings

Fig. 1 is a transmission electron microscope photograph of a composite body according to an example 1.
Fig. 2 is a transmission electron microscope photograph of the composite body according to the example 1.
Fig. 3 is a transmission electron microscope photograph of a composite body according to an example 2.
Fig. 4 is a transmission electron microscope photograph of a composite body according to an example 3.
Fig. 5 is a transmission electron microscope photograph of a composite body according to an example 4.
Fig. 6 is a transmission electron microscope photograph of a composite body according to an example 5.
Fig. 7 is a transmission electron microscope photograph of a composite body according to an example 6.

### Description of Embodiments

Hereinafter, a composite body and a method for producing a composite body according to the present invention are described with reference to embodiment and examples. The overlapping descriptions are omitted when appropriate. In the case where a numerical value range is expressed using "to" between two numerical values, such two numerical values also fall within the numerical value range.

A composite body according to the embodiment of the present invention has: a base material; and a ceramic layer which is coated onto at least a portion of a surface of the base material. The base material contains a hydrophilic polymer. As the hydrophilic polymer, protein, peptide, polysaccharide, a synthetic polymer or the like can be exemplified. However, the hydrophilic polymer is not particularly limited. The hydrophilic polymer is preferably protein or polysaccharide. When the hydrophilic polymer is protein, the hydrophilic polymer is preferably at least one of gelatin and a bovine serum albumin (BSA). When the hydrophilic polymer is polysaccharide, the hydrophilic polymer is preferably at least one of pectin, a hyaluronic acid, and carboxymethyl cellulose. As a hydrophilic functional group which the base material contains, a carboxyl group, a sulfo group, a phosphate group and the like can be exemplified. However, the hydrophilic functional group is not limited to such groups.

The ceramic layer contains crystals having a laminated structure. The ceramic layer may be formed of crystals having a laminated structure. The crystals having the laminated structure may preferably contain calcium phosphate. Calcium phosphate is a salt formed of calcium ions (Ca²⁺), phosphate ions (PO₄³⁻) or diphosphoric acid ions (P₂O₇⁴⁻). In this embodiment, as calcium phosphate contained in the ceramic layer, for example, calcium dihydrogen phosphate (Ca(H₂PO₄)₂), calcium dihydrogen phosphate monohydrate (Ca(H₂PO₄)₂.H₂O), calcium hydrogen phosphate (CaHPO₄), calcium hydrogen phosphate dihydrate (CaHPO₄.2H₂O), tricalcium phosphate (Ca₃(PO₄)₂), octacalcium phosphate (Ca₈H₂(PO₄)₆.5H₂O), hydroxyapatite (Ca₁₀(PO₄)₆(OH)₂) and the like are named.

Calcium phosphate preferably contains hydroxyapatite. The laminated structure of this hydroxyapatite is preferably formed by laminating hydroxyapatite toward the outside of the base material in a c axis direction. By laminating hydroxyapatite in the c axis direction toward the outside of the base material, the ceramic layer can be strengthened and hence, the base material can be stably held. The laminated structure preferably exists over the entire surface of the ceramic layer. For example, an outermost shell made of ceramic crystals is preferably made of hydroxyapatite having a regular crystal structure.

In the ceramic layer according to the embodiment, at least a portion of calcium or phosphoric acid contained in calcium phosphate is chemically bonded to a hydrophilic functional group of a surface of the base material. That is, calcium or phosphoric acid existing in an inner portion of the ceramic layer and a functional group derived from anion group existing on a surface of the base material are chemically bonded to each other. Accordingly, the ceramic layer according to the embodiment is minimally peeled off from the base material and hence, the ceramic layer is minimally broken.

In the case where a thickness of the ceramic layer according to the embodiment is 0.3 nm to 50 nm, preferably 1 nm to 20 nm, and more preferably 1 nm to 10 nm, the ceramic layer according to the embodiment is suitable for applications such as imparting hardness to an organic base material or imparting chemical resistance to the organic base material. This is because an absolute amount of the ceramic layer is small and hence, it is possible to impart hardness and chemical resistance to the organic base material without reducing an advantage of the organic base material that a weight of the base material is small. It is also because by setting a thickness of the ceramic layer smaller than a wavelength of a visible light, it is possible to suppress the occurrence of a state where an optical property of the base material is damaged. The composite body according to the embodiment may be a core-shell particle where a base material forms a core and a ceramic layer forms a shell.

A method for producing a composite body according to the embodiment of the present invention is a method for producing a composite body having a base material and a ceramic layer which is coated onto at least a portion of a surface of the base material, wherein the method has a step of mixing and stirring a dispersion liquid in which the base material is dispersed, a first solution containing calcium ions, and a second solution containing phosphate ions. A dispersion medium of the dispersion liquid and solvents of the first solution and the second solution are preferably water. This is because the preparation and handling of the dispersion liquid, the first solution, and the second solution can be performed easily. The base material may contain a hydrophilic polymer.

In the dispersion liquid, the base material is dispersed in a shape suitable for being coated with ceramic crystals. The dispersion liquid can be prepared by mixing the base material and a dispersion medium. When necessary, the dispersion liquid may be prepared by further stirring the base material and the dispersion medium using a propeller type stirrer, a magnetic stirrer or the like. The base material may be dispersed in the first solution, or the base material may be dispersed in the second solution. As the first solution which is an aqueous solution, a calcium nitrate tetrahydrate aqueous solution, a calcium chloride aqueous solution, a calcium chloride monohydrate aqueous solution, a calcium chlorate dihydrate aqueous solution, a calcium perchlorate aqueous solution, a calcium bromide aqueous solution, a calcium acetate aqueous solution and the like can be exemplified. However, the first solution is not limited to such aqueous solutions.

Concentration of calcium ions in the first solution is preferably 2 mol/L or below and preferably 1×10⁻⁶ mol/L to 2×10⁻¹ mol/L. This is because calcium phosphate can be regularly precipitated on a surface of the base material. As the second solution which is an aqueous solution, a diammonium hydrogen phosphate aqueous solution, an ammonium dihydrogen phosphate aqueous solution, a disodium hydrogen phosphate aqueous solution, a sodium dihydrogen phosphate monohydrate aqueous solution, a sodium dihydrogen phosphate dihydrate aqueous solution, a potassium phosphate aqueous solution, a dipotassium hydrogenphosphate aqueous solution, a potassium dihydrogenphosphate aqueous solution, a phosphoric acid aqueous solution and the like can be exemplified. However, the second solution is not limited to such aqueous solutions.

The method for producing a composite body according to this embodiment may adopt (1) a step of mixing and stirring a dispersion liquid in which the base material is dispersed and which contains calcium ions and the second solution or (2) a step of mixing and stirring a dispersion liquid in which the base material is dispersed and which contains phosphate ions and the first solution instead of the step of mixing and stirring the dispersion liquid in which the base material is dispersed, the first solution, and the second solution. The order of mixing the dispersion liquid in which the base material is dispersed, the first solution, and the second solution is not particularly limited. The dispersion liquid in which the base material is dispersed, the first solution, and the second solution are preferably stirred under an environment where pH is 9 or more.

A ratio of an amount of substance of calcium ions in the first solution with respect to an amount of substance of phosphate ions in the second solution, that is, a so-called molar ratio, is preferably 0.8 to 20. This is because a molar ratio of an amount of substance of calcium ions with respect to an amount of substance of phosphate ions is 1.7 in case of hydroxyapatite to be precipitated and hence, hydroxyapatite can be efficiently precipitated when the molar ratio of an amount of substance of calcium ions in the first solution with respect to an amount of substance of phosphate ions in the second solution is substantially at the same level. A method of stirring the dispersion liquid and the solutions is not particularly limited. For example, the solutions can be stirred by using a stirring unit such as a propeller type stirrer or a magnetic stirrer, for example. A stirring time is, for example, 10 minutes to 120 hours. The stirring time is preferably 1 hour to 72 hours for accelerating the precipitation of calcium phosphate on a surface of the base material.

Through such a step of mixing and stirring the dispersion liquid, the first solution, and the second solution, it is possible to obtain a dispersion body of a composite body which contains a base material and ceramic crystals formed on a surface of the base material. By applying the solid liquid separation to such a dispersion liquid by filtering, centrifugal separation and freeze drying or the like, the composite body can be separated in a single form. The method for producing a composite body according to this embodiment can be performed at a normal temperature and at a normal pressure and hence, the degradation of the base material can be suppressed. Further, a burden imposed on an environment can be also reduced. When necessary, it is also possible to heat the dispersion liquid in the method of manufacturing a composite body.

### [Example]

Next, the present invention is specifically described with reference to examples. However, the present invention is not limited to such examples.

### [Example 1]

A dispersion liquid was prepared by dispersing 0.005g of gelatin (made by Nippi, Incorporated, HMG-BP) in 65 mL of distilled water. 10 mL of calcium acetate aqueous solution having concentration of 0.018 mol/L was added to the dispersion liquid. The dispersion liquid was stirred for 5 minutes at 300 rpm using a magnetic stirrer. 25 mL of diammonium hydrogen phosphate aqueous solution having concentration of 0.003 mol/L was further added to the dispersion liquid, and the dispersion liquid was stirred. Then, the dispersion liquid was stirred for 24 hours at 300 rpm using a magnetic stirrer. As a result, an aqueous dispersion liquid containing composite bodies where calcium phosphate crystals were coated onto a surface of gelatin was obtained. Solid liquid separation was applied to the aqueous dispersion liquid by centrifugal separation and, thereafter, freeze drying was applied to the aqueous dispersion liquid thus obtaining core-shell particles.

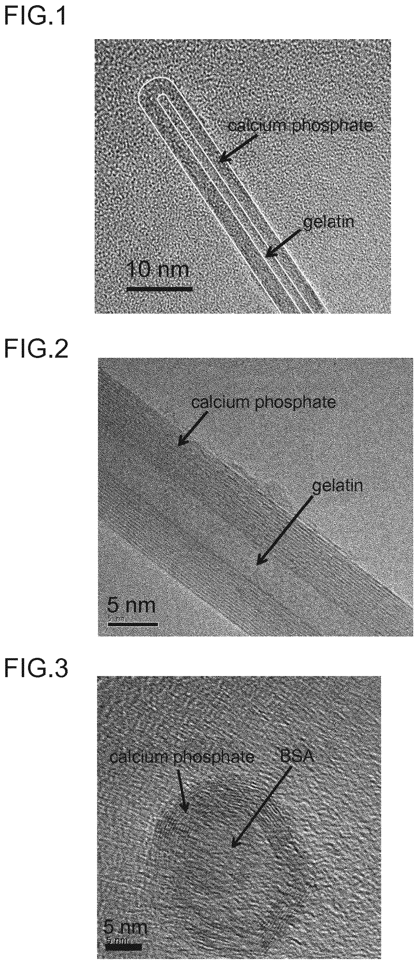

A transmission electron microscope photograph of the core-shell particle is shown in Fig. 1. It was confirmed from this photograph that a shell having a thickness of approximately 5 nm and made of ceramic crystals is uniformly coated onto a surface of a gelatinous fiber which is a core. Fig. 2 is a transmission electron microscope photograph obtained by observing the core-shell particle with a higher magnification. It was confirmed from a grid image obtained by the transmission electron microscope that hydroxyapatite was laminated toward an outside of the shell in a c axis direction.

### [Example 2]

50 mg of bovine serum albumin (BSA) (made by Sigma-Aldrich) was added to 100 mL of calcium acetate aqueous solution having concentration of 0.010 mol/L. The calcium acetate aqueous solution was stirred for 5 minutes at 300 rpm using a magnetic stirrer. 100 mL of diammonium hydrogen phosphate aqueous solution having concentration of 0.006 mol/L was further dropped into the calcium acetate aqueous solution and the calcium acetate aqueous solution was stirred. Then, the calcium acetate aqueous solution was stirred for 3 hours at 300 rpm using a magnetic stirrer. As a result, an aqueous dispersion liquid containing composite bodies where calcium phosphate crystals were coated onto a surface of BSA was obtained. Filtering is applied to the aqueous dispersion liquid and, thereafter, freeze drying was applied to the aqueous dispersion liquid thus obtaining core-shell particles.

Fig. 3 is a transmission electron microscope photograph of the core-shell particle. It was confirmed from this photograph that a shell having a thickness of approximately 5 nm and made of ceramic crystals is uniformly coated onto a surface of a BSA which is a core. It was also confirmed from a grid image obtained by a transmission electron microscope that hydroxyapatite was laminated toward an outside of the shell in a c axis direction.

### [Example 3]

A dispersion liquid was prepared by dispersing 0.05g of pectin (made by FUJIFILM Wako Pure Chemical Corporation, 164-00552) in 160 mL of distilled water. 20 mL of calcium acetate aqueous solution having concentration of 0.080 mol/L was added to the dispersion liquid. The dispersion liquid was stirred for 5 minutes at 300 rpm using a magnetic stirrer. 25 mL of diammonium hydrogen phosphate aqueous solution having concentration of 0.048 mol/L was further added, and the dispersion liquid was stirred. Then, the dispersion liquid was stirred for 24 hours at 300 rpm using a magnetic stirrer. As a result, an aqueous dispersion liquid containing core-shell particles where hydroxyapatite crystals were formed on a surface of pectin was obtained. Solid liquid separation was applied to the aqueous dispersion liquid by centrifugal separation and, thereafter, freeze drying was applied to a solid material thus obtaining core-shell particles having a width of approximately 20 nm and a length of approximately 500 nm.

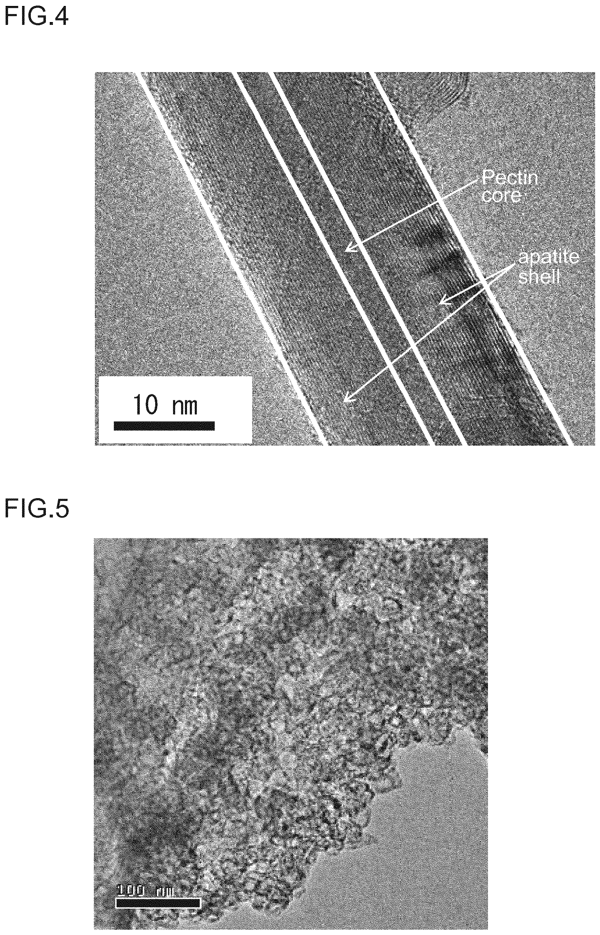

Fig. 4 is a transmission electron microscope photograph of the core-shell particle. It was confirmed from this photograph that a shell having a thickness of approximately 10 nm and made of ceramic crystals was uniformly coated onto a surface of pectin which is a core. It was also confirmed from a grid image obtained by a transmission electron microscope that hydroxyapatite was laminated toward an outside of the shell in a c axis direction.

### [Example 4]

A dispersion liquid was prepared by dispersing O.lg of sodium hyaluronate (made by FUJIFILM Wako Pure Chemical Corporation, 089-10343) in 200 mL of distilled water. 2 mL of calcium acetate aqueous solution having concentration of 2.00 mol/L was added to the dispersion liquid. The dispersion liquid was stirred for 5 minutes at 300 rpm using a magnetic stirrer. 2 mL of diammonium hydrogen phosphate aqueous solution having concentration of 1.20 mol/L was further added to the dispersion liquid, and the dispersion liquid was stirred. Then, the dispersion liquid was stirred for 24 hours at 300 rpm using a magnetic stirrer. As a result, an aqueous dispersion liquid containing core-shell particles where hydroxyapatite crystals were formed on a surface of a hyaluronic acid was obtained. Solid liquid separation was applied to the aqueous dispersion liquid by centrifugal separation and, thereafter, freeze drying was applied to the solid material thus obtaining core-shell particles having a diameter of approximately 20 nm.

Fig. 5 is a transmission electron microscope photograph of the core-shell particle. It was confirmed from this photograph that the core-shell particle had the structure where a hydroxyapatite crystal shell having a thickness of approximately 5 nm was coated onto a periphery of a core which was made of hyaluronic acid fibers formed into a spherical shape. Further, it was confirmed that a crystal plane of a surface of the core-shell particle was a c plane.

### [Example 5]

A dispersion liquid was prepared by dispersing O.lg of carboxymethyl cellulose (made by FUJIFILM Wako Pure Chemical Corporation, 039-01335) in 200 mL of distilled water. 2 mL of calcium acetate aqueous solution having concentration of 0.80 mol/L was added to the dispersion liquid. The dispersion liquid was stirred for 5 minutes at 300 rpm using a magnetic stirrer. 2 mL of diammonium hydrogen phosphate aqueous solution having concentration of 0.48 mol/L was further added to the dispersion liquid, and the dispersion liquid was stirred. Then, the dispersion liquid was stirred for 24 hours at 300 rpm using a magnetic stirrer. As a result, an aqueous dispersion liquid containing core-shell particles where hydroxyapatite crystals were formed on a surface of carboxymethyl cellulose was obtained. Solid liquid separation was applied to the aqueous dispersion liquid by centrifugal separation and, thereafter, freeze drying was applied to the solid material thus obtaining fibrous core-shell particles.

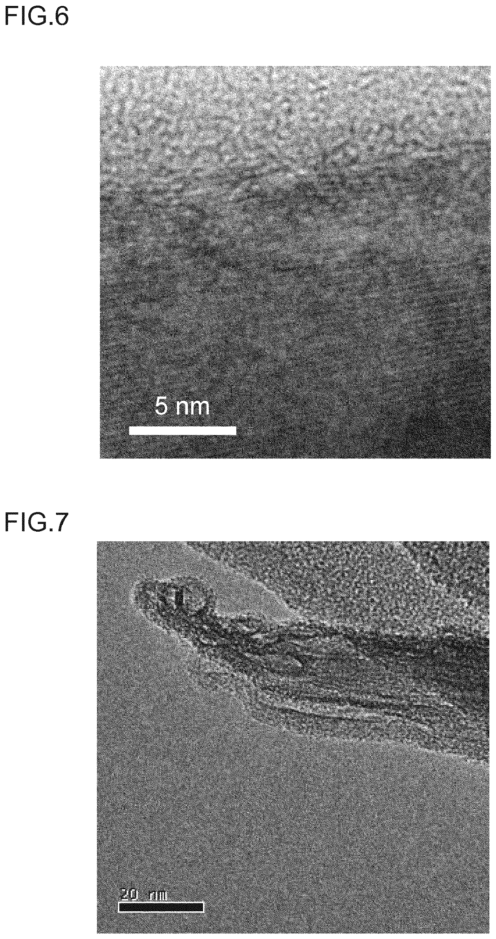

Fig. 6 is a transmission electron microscope photograph of the core-shell particle. It was confirmed from this photograph that the core-shell particle had the structure where a hydroxyapatite crystal shell having a thickness of approximately 10 nm was coated onto carboxymethyl cellulose fibers which formed a core. Further, it was confirmed that a crystal plane of a surface of the core-shell particle was a c plane.

### [Example 6]

In this example 6, fibrous core-shell particles were obtained in the same manner as the example 5 except for a point that a calcium acetate aqueous solution having concentration of 2.00 mol/L and a diammonium hydrogen phosphate aqueous solution having concentration of 1.20 mol/L were used. Fig. 7 is a transmission electron microscope photograph of the core-shell particle. It was confirmed from this photograph that the core-shell particle had the structure where carboxymethyl cellulose fibers each having a width of approximately 5 nm formed a core, and a hydroxyapatite crystal shell having a thickness of approximately 5 nm was coated onto a periphery of the core. Further, it was confirmed that a crystal plane of a surface of the core-shell particle was a c plane.

### [Example 7]

A dispersion liquid was prepared by dispersing O.lg of pectin (made by FUJIFILM Wako Pure Chemical Corporation, 164-00552) in 160 mL of distilled water. 20 mL of calcium acetate aqueous solution having concentration of 0.020 mol/L was added to the dispersion liquid. The dispersion liquid was stirred for 5 minutes at 300 rpm using a magnetic stirrer. 20 mL of diammonium hydrogen phosphate aqueous solution having concentration of 0.012 mol/L was further added to the dispersion liquid, and the dispersion liquid was stirred. Then, the dispersion liquid was stirred for 72 hours at 300 rpm using a magnetic stirrer. As a result, an aqueous dispersion liquid containing core-shell particles where hydroxyapatite crystals were formed on a surface of pectin was obtained. Desalination was applied to the aqueous dispersion liquid using a permeable membrane and, thereafter, freeze drying was applied to the aqueous dispersion liquid thus obtaining sheet-like core-shell particles.

It was confirmed from a transmission electron microscope photograph of the core-shell particle that the core-shell particle had the sheet structure where fibrous composite bodies in each of which pectin formed a core and a hydroxyapatite crystal shell having a thickness of approximately 5 nm was coated onto a periphery of the core were linked to each other in a lateral direction. Further, it was confirmed that a crystal plane of a surface of the core-shell particle was a c plane.

### [Example 8]

In this example 8, sheet-like core-shell particles were obtained in the same manner as the example 7 except for a point that a calcium acetate aqueous solution having concentration of 0.040 mol/L and a diammonium hydrogen phosphate aqueous solution having concentration of 0.024 mol/L were used and a point that stirring using a magnetic stirrer was changed from 72 hours to 24 hours. It was confirmed from a transmission electron microscope photograph of the core-shell particle that the core-shell particle had the sheet structure where fibrous composite bodies in each of which pectin formed a core and a hydroxyapatite crystal shell having a thickness of approximately 3 nm was coated onto a periphery of the core are linked to each other in a lateral direction. Further, it was confirmed that a crystal plane of a surface of the core-shell particle was a c plane.

### [Example 9]

In this example 9, sheet-like core-shell particles were obtained in the same manner as the example 8 except for a point that a calcium acetate aqueous solution having concentration of 0.080 mol/L and a diammonium hydrogen phosphate aqueous solution having concentration of 0.048 mol/L were used. It was confirmed from a transmission electron microscope photograph of the core-shell particle that the core-shell particle had the sheet structure where fibrous composite bodies in each of which pectin formed a core and a hydroxyapatite crystal shell having a thickness of approximately 5 nm was coated onto a periphery of the core were linked to each other in a lateral direction. Further, it was confirmed that a crystal plane of a surface of the core-shell particle was a c plane.

### [Example 10]

In this example 10, sheet-like core-shell particles were obtained in the same manner as the example 9 except for a point that a calcium acetate aqueous solution having concentration of 0.80 mol/L and 20 mL of a diammonium hydrogen phosphate aqueous solution having concentration of 0.48 mol/L were used and a point that solid liquid separation was applied to an aqueous dispersion liquid containing core-shell particles by centrifugal separation. It was confirmed from a transmission electron microscope photograph of the core-shell particle that the core-shell particle had the sheet structure where fibrous composite bodies in each of which pectin formed a core and a hydroxyapatite crystal shell having a thickness of approximately 10 nm was coated onto a periphery of the core were linked to each other in a lateral direction. Further, it was confirmed that a crystal plane of a surface of the core-shell particle was a c plane.

### [Example 11]

A dispersion liquid was prepared by dispersing O.lg of carboxymethyl cellulose (made by FUJIFILM Wako Pure Chemical Corporation, 039-01335) in 160 mL of distilled water. 20 mL of calcium acetate aqueous solution having concentration of 0.020 mol/L was added to the dispersion liquid. The dispersion liquid was stirred for 5 minutes at 300 rpm using a magnetic stirrer. 20 mL of diammonium hydrogen phosphate aqueous solution having concentration of 0.012 mol/L was further added to the dispersion liquid, and the dispersion liquid was stirred. Then, the dispersion liquid was stirred for 24 hours at 300 rpm using a magnetic stirrer. As a result, an aqueous dispersion liquid containing core-shell particles where hydroxyapatite crystals were formed on a surface of carboxymethyl cellulose was obtained. Desalination was applied to the aqueous dispersion liquid using a permeable membrane and, thereafter, freeze drying was applied to the aqueous dispersion liquid thus obtaining sheet-like core-shell particles.

It was confirmed from a transmission electron microscope photograph of the core-shell particle that the core-shell particle had the sheet structure where fibrous composite bodies in each of which carboxymethyl cellulose fibers formed a core and a hydroxyapatite crystal shell having a thickness of approximately 2 nm was coated onto a periphery of the core were linked to each other in a lateral direction. Further, it was confirmed that a crystal plane of a surface of the core-shell particle was a c plane.

### [Example 12]

A dispersion liquid was prepared by dispersing 0.5g of carboxymethyl cellulose (made by FUJIFILM Wako Pure Chemical Corporation, 039-01335) in 180 mL of distilled water. 20 mL of calcium acetate aqueous solution having concentration of 0.020 mol/L was added to the dispersion liquid. The dispersion liquid was stirred for 5 minutes at 300 rpm using a magnetic stirrer. 20 mL of diammonium hydrogen phosphate aqueous solution having concentration of 0.012 mol/L was further added to the dispersion liquid, and the dispersion liquid was stirred. Then, the dispersion liquid was stirred for 72 hours at 300 rpm using a magnetic stirrer. As a result, an aqueous dispersion liquid containing core-shell particles where hydroxyapatite crystals were formed on a surface of carboxymethyl cellulose was obtained. Solid liquid separation was applied to the aqueous dispersion liquid by centrifugal separation and, thereafter, freeze drying was applied to a solid material thus obtaining sheet-like core-shell particles.

It was confirmed from a transmission electron microscope photograph of the core-shell particle that the core-shell particle had the sheet structure where fibrous composite bodies in each of which carboxymethyl cellulose fibers formed a core and a hydroxyapatite crystal shell having a thickness of approximately 5 nm was coated onto a periphery of the core were linked to each other in a lateral direction. Further, it was confirmed that a crystal plane of a surface of the core-shell particle was a c plane.

### [Example 13]

In this example 13, sheet-like core-shell particles were obtained in the same manner as the example 12 except for a point that the dispersion liquid was stirred for 72 hours at 300 rpm using a magnetic stirrer and, thereafter, a temperature of the aqueous dispersion liquid was elevated from a room temperature to 60°C. It was confirmed from a transmission electron microscope photograph of the core-shell particle that the core-shell particle had the sheet structure where fibrous composite bodies in each of which carboxymethyl cellulose fibers formed a core and a hydroxyapatite crystal shell having a thickness of approximately 10 nm was coated onto a periphery of the core were linked to each other in a lateral direction. Further, it was confirmed that a crystal plane of a surface of the core-shell particle was a c plane.

### [Example 14]

In this example 14, sheet-like core-shell particles were obtained in the same manner as the example 8 except for a point that a calcium acetate aqueous solution having concentration of 0.020 mol/L and a diammonium hydrogen phosphate aqueous solution having concentration of 0.012 mol/L were used. It was confirmed from a transmission electron microscope photograph of the core-shell particle that the core-shell particle had the sheet structure where fibrous composite bodies in each of which pectin formed a core and a hydroxyapatite crystal shell having a thickness of approximately 1 nm was coated onto a periphery of the core were linked to each other in a lateral direction. Further, it was confirmed that a crystal plane of a surface of the core-shell particle was a c plane.

### Industrial Applicability

The composite body according to the present invention can be used for imparting hardness, chemical resistance and the like to an organic base material.

## Claims

1. A composite body comprising:
a base material containing a hydrophilic polymer; and
a ceramic layer which is coated onto at least a portion of a surface of the base material, wherein
the ceramic layer contains crystals having a laminated structure.

2. The composite body according to claim 1, wherein the ceramic layer is formed of crystals having a laminated structure.

3. The composite body according to claim 1 or 2, wherein the crystals having the laminated structure contain calcium phosphate.

4. The composite body according to claim 3, wherein the calcium phosphate contains hydroxyapatite.

5. The composite body according to claim 4, wherein the hydroxyapatite is laminated toward an outside of the base material in a c axis direction.

6. The composite body according to any one of claims 1 to 5, wherein a thickness of the ceramic layer is 0.3 nm to 50 nm.

7. A ceramic crystal shell according to claim 6, wherein a thickness of the ceramic layer is 1 nm to 20 nm.

8. The ceramic crystal shell according to claim 7, wherein a thickness of the ceramic layer is 1 nm to 10 nm.

9. The composite body according to any one of claims 1 to 8, wherein the composite body is a core-shell particle where the base material forms a core and the ceramic layer forms a shell.

10. The composite body according to any one of claims 1 to 9, wherein the base material has a sheet shape.

11. The composite body according to any one of claims 1 to 10, wherein the hydrophilic polymer is protein.

12. The composite body according to claim 11, wherein the protein is gelatin or a bovine serum albumin (BSA).

13. The composite body according to any one of claims 1 to 10, wherein the hydrophilic polymer is polysaccharide.

14. The composite body according to claim 13, wherein the polysaccharide is pectin, a hyaluronic acid, or carboxymethyl cellulose.

15. A method for producing a composite body having a base material and a ceramic layer which is coated onto at least a portion of a surface of the base material, the method comprising a step of mixing and stirring a dispersion liquid in which the base material is dispersed, a first solution containing calcium ions, and a second solution containing phosphate ions.

16. The method for producing a composite body according to claim 15, wherein the base material contains a hydrophilic polymer.

17. The method for producing a composite body according to claim 15 or 16, wherein a dispersion medium of the dispersion liquid and solvents of the first solution and the second solution are water.

18. The method for producing a composite body according to any one of claims 15 to 17, wherein concentration of calcium ions in the first solution is 2 mol/L or below.

19. The method for producing a composite body according to any one of claims 15 to 18, wherein, in the step of mixing and stirring the dispersion liquid, the first solution, and the second solution, the dispersion liquid, the first solution, and the second solution are stirred under an environment where pH is 9 or more.
